(19) Europäisches Patentamt
European Patent Office
Office européen des brevets

(11) EP 1 847 264 B1

(12) **EUROPEAN PATENT SPECIFICATION**

(45) Date of publication and mention
of the grant of the patent:
**03.05.2017 Bulletin 2017/18**

(51) Int Cl.:
**A61K 31/138** (2006.01)          **A61K 9/70** (2006.01)
**A61K 47/32** (2006.01)          **A61P 9/12** (2006.01)

(21) Application number: **06711700.2**

(22) Date of filing: **16.01.2006**

(86) International application number:
**PCT/JP2006/300419**

(87) International publication number:
**WO 2006/080199 (03.08.2006 Gazette 2006/31)**

(54) **Bisoprolol patch**

Bisoprolol Pflaster

Patch contenant du Bisoprolol

(84) Designated Contracting States:
**AT BE BG CH CY CZ DE DK EE ES FI FR GB GR HU IE IS IT LI LT LU LV MC NL PL PT RO SE SI SK TR**

(30) Priority: **31.01.2005 JP 2005024049**

(43) Date of publication of application:
**24.10.2007 Bulletin 2007/43**

(73) Proprietor: **Hisamitsu Pharmaceutical Co., Inc.**
**Tosu-shi**
**Saga 841-0017 (JP)**

(72) Inventors:
• **AMANO, Satoshi**
**Tsukuba-shi, Ibaraki 305-0856 (JP)**
• **HONMA, Sachiko**
**Tsukuba-shi, Ibaraki 305-0856 (JP)**
• **TATEISHI, Tetsuro**
**Tsukuba-shi, Ibaraki 305-0856 (JP)**

(74) Representative: **Dr. Gassner & Partner mbB**
**Marie-Curie-Str. 1**
**91052 Erlangen (DE)**

(56) References cited:
**EP-A1- 1 462 121          EP-A1- 1 961 417**
**WO-A1-00/61120          WO-A1-95/18603**
**WO-A1-02/069942          JP-A- 11 302 161**
**JP-A- 2003 313 122          JP-A- 2004 010 525**
**JP-A- 2004 083 523**

**Description**

[Technical Field]

The invention relates to a patch comprising Bisoprolol which is a ß-blocker, said patch containing a backing and a Bisoprolol-containing pressure-sensitive adhesive layer comprising a base, wherein the Bisoprolol is contained in 10-15 mass % based on the total amount of the base, wherein the base comprises an acrylic polymer, and wherein a skin penetration rate of Bisoprolol after 24 hours is 15 to 60% of a maximum skin penetration rate thereof.

[Background Art]

As administration methods for drugs, an oral administration using tablets, capsules, syrups and the like has been made in many drugs. However, in case of the oral administration, there were drawbacks that it lacked in duration of the effect, an unnecessary high blood concentration was recognized for a while after the administration, whereby a side effect occurred easily, and the like.

Bisoprolol is high in $ß_1$-receptor selectivity and a ß-blocker which does not have an intrinsic sympathomimetic activity and a membrane stabilizing activity. At present, in clinical treatment it is used mainly in an oral formulation as a therapeutic drug for hypertension, angina pectoris, ventricular premature beat and etc. Although Bisoprolol is relatively little in the effect to bronchus due to the high $ß_1$ selectivity, there are cases that symptoms such as bradycardia, dizziness and physical weariness occur, and there were problems in the point of stabilization of the concentration in blood and sustainability of the effect.

In the meantime, the percutaneous absorption type formulations are expected to have merits such as reduction of administration frequency, improvement of compliance and simplicity of administration as well as its discontinuation, and are known to be useful particularly in aged and infantile patients. As a percutaneous formulation which makes Bisoprolol the active ingredient, a patch was reported in which a pressure-sensitive adhesive layer containing an acrylic adhesive polymer, in which alkyl (meth)acrylate was polymerized with a monomer polymerizable to this and containing neither of a carboxyl group nor a sulfo group, and Bisoprolol or an pharmacologically acceptable salt thereof was set on one side of a backing (patent document 1). However, although this patch attained almost constant and stable skin penetration rate (Fig. 1 and Fig. 2 in patent document 1), there were inconveniences such as difficulty to obtain an effective blood concentration for therapy or necessity to take much time till the blood concentration when repeating administration was stabilized.

Patent document 1: JP, A, 2003-313122

Prior art exists which relate to patches and include EP 14622121 which describes a patch comprising a laminated backing layer and a drug-containing pressure-sensitive adhesive layer, wherein the laminated backing layer consists of a knitted fabric laminated on one side of a polyester film, and the drug-containing pressure-sensitive adhesive layer is laminated on the other side of the polyester film, wherein the knitted fabric has a 30% longitudinal modulus of 0.1kg/5 cm to 20 kg/5 cm and a 30% lateral modulus of not more than 10kg/5 cm, and a 30% longitudinal modulus/30% lateral modulus ratio of not less than 2. WO9518603 describes a blend of at least three polymers, including a soluble polyvinylpyrrolidone, in combination with a drug provides a pressure-sensitive adhesive composition for a transdermal drug delivery system in which the drug is delivered from the pressure-sensitive adhesive composition through the dermis when the pressure-sensitive adhesive composition is in contact with human skin. The soluble polyvinylpyrrolidone is disclosed as increasing the solubility of the drug without negatively affecting the adhesivity of the composition or the rate of drug delivery from the pressure-sensitive adhesive composition.

JP2004083523 discloses a patch composed of a substrate and a pressure-sensitive adhesive layer which is placed on the substrate and contains a drug and a pressure-sensitive adhesive base material containing a styrene/isoprene/styrene block copolymer, a 2-ethylhexyl acrylate/vinyl acetate copolymer, and a basic nitrogen- containing polymer containing basic nitrogen and nontacky at normal temperature.

[Disclosure of the Invention]

[Problem to Be Solved by the Invention]

The invention makes it an object to provide a patch containing Bisoprolol and/or a pharmaceutically acceptable salt thereof, wherein it shows a little difference between the maximum level of the concentration in blood and the minimum level thereof in repeated administration, and therefore, scarcely exhibits serious side effects owing to an excess hypotension, and moreover, achieves the quick development of the drug effect owing to the stabilization of the concentration in blood within a short time.

[Means for Solving Problem]

The inventors found that by making the claimed patch containing Bisoprolol and/or a pharmaceutically acceptable salt thereof a formulation with a specific ratio between the level of the skin penetration rate of Bisoprolol after 24 hours and the maximum level thereof, it was possible to provide a Bisoprolol patch in which in a repeated administration (preferably once a day) the development of the drug effect was quick, a side effect was scarce and the stable drug effect was obtained owing to a little difference in the change of the concentration in blood, and moreover, skin irritation was scarce.

Namely, the invention relates to the claimed Bisoprolol patch, wherein the skin penetration rate of Bisoprolol after 24 hours is 15 to 60% of the maximum skin penetration rate thereof.

In addition, the invention relates to the above Bisoprolol patch, wherein the maximum skin penetration rate of Bisoprolol is obtained within 12 hours during administration.

Further, the invention relates to the above Bisoprolol patch, wherein the skin diffusion coefficient of Bisoprolol is $2.5 \times 10E\text{-}8 \sim 6 \times 10E\text{-}8$ cm$^2$/sec.

In addition, the invention relates to the above Bisoprolol patch, wherein Bisoprolol and/or a pharmaceutically acceptable salt thereof are contained in 10-15 mass % based on the total amount of a base of the claimed patch.

Further, the invention relates to the above Bisoprolol patch, wherein the base of the patch formulation comprises an acrylic polymer and one or more selected from a styrene-isoprene-styrene block copolymer, polyisoprene, and a silicone type polymer.

Furthermore, the invention relates to the above Bisoprolol patch, wherein the base of the patch formulation comprises an acrylic polymer and a rubber polymer.

[Effect of the Invention]

By a Bisoprolol patch of the invention, it is possible to provide a safe patch, wherein it shows a little difference between the maximum level of the concentration in blood and the minimum level thereof even in repeated administration necessary for therapy of the essential hypertension, and therefore, scarcely exhibits serious side effects due to an excess hypotension, and moreover, achieves the quick development of the drug effect owing to the stabilization of the concentration in blood within a short time, and moreover, skin irritation is scarce.

[Best Embodiment for Carrying out the Invention]

In the following, the patch of the invention is illustrated in more detail.

The patch of the invention indicates a patch containing at least a backing and a pressure-sensitive adhesive layer composition and comprises an external patch of reservoir type and an external patch of matrix type which are called generally. Comparing the external patch of reservoir type and the external patch of matrix type, generally, the external patch of matrix type, in which a composition of a pressure-sensitive adhesive layer having a self adhesive force adheres directly to the skin, is more excellent in adhesiveness and is also excellent in penetration of a drug to the skin, and therefore, in the following the patch of the invention is mainly explained taking a patch of matrix type as an example, though it is not limited this.

In addition, a hydrophobic polymer in the specification is a polymer using an organic solvent or an organic solvent mixture as solvents for polymer when preparing a pressure-sensitive adhesive layer of the patch.

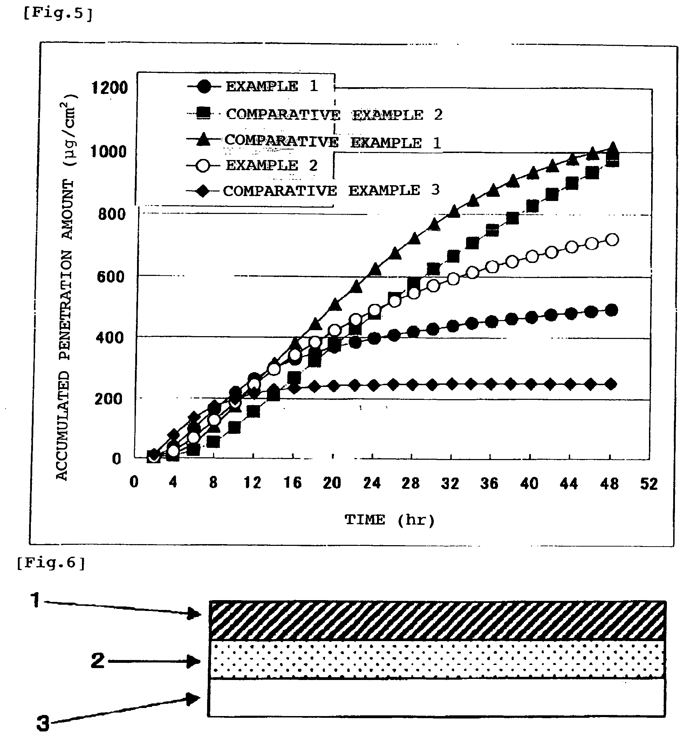

The patch of the invention is typically a form which consists of a hydrophobic matrix (pressure-sensitive adhesive layer) containing a drug (Bisoprolol and/or a pharmaceutically acceptable salt thereof) as is shown in Fig.6, and a backing on its back. Although it is preferable that this pressure-sensitive adhesive layer has an adhesive force to maintain an effective area on a surface of the skin without problem for therapy for at least 12 or more hours, in a case that it is difficult, it is possible to use a sheet type cover which has a larger area compared with a layer containing the drug and also has an adhesive force.

Bisoprolol which can be used in the invention may be a free form or its salt. In case of using the salt, it is not particularly limited if it is a pharmaceutically acceptable salt, however, preferably fumarate, hydrochloride, sulfonate, mesylate, citrate, tartarate, maleate or acetate. Among these, Bisoprolol hemifumarate is more preferable.

Bisoprolol used in the invention is contained in 10-15 mass %, based on the total amount of a patch base from the viewpoint of skin penetration and physical properties of a formulation.

The maximum skin penetration rate of Bisoprolol in the patch of the invention is obtained within 12 hours during administration, preferably within 10 hours, and more preferably within 8 hours owing to the stabilization of the concentration in blood within a short time.

In addition, the skin penetration rate of Bisoprolol after 24 hours in the patch of the invention is 15 to 60% of the maximum skin penetration rate thereof, preferably 20 to 40%, and more preferably 20 to 30% from the viewpoint of a little difference between the maximum level of the concentration in blood and the minimum level thereof and the stabilization in the concentration in blood within a short time.

Here, the skin penetration rate of Bisoprolol (the maximum level) can be obtained by a human skin penetration test. As for a specific test method, a human skin (the abdomen) for test use, which was removed in thickness of about 500 $\mu$m from the skin's cornified-layer side, is used. Each formulation is attached to the skin's cornified-layer side and the dermal side is placed to the receptor-layer side to mount the skin on a flow-through type diffusion cell. Further, using pH7.4 phosphate buffer saline in the receptor layer, warm water is circulated around the outer part to make the temperature of the skin surface $32 \pm 1°C$. Samplings are carried out at every 2 hours. As to each of the receptor solution obtained, the flow volume is accurately measured, the drug concentration is measured by high-performance liquid chromatography, the penetration rate per hour is calculated by measured values of the flow volume and the drug concentration, and the maximum skin penetration rate can be determined.

The skin diffusion coefficient of Bisoprolol in the patch of the invention is $2.5 \times 10E\text{-}8 \sim 6 \times 10E\text{-}8$ cm$^2$/sec, preferably $3 \times 10E\text{-}8 \sim 6 \times 10E\text{-}8$ cm$^2$/sec, and more preferably $3 X 10E\text{-}8 \sim 5 \times 10E\text{-}8$ cm$^2$/sec from the viewpoint that after the start of administration a stable concentration in blood is obtained within a relatively short time, and also, a little difference between the maximum level of the concentration in blood and the minimum level thereof is shown and skin irritation is scarcely exhibited to give a safe formulation.

Here, the skin diffusion coefficient D can be obtained by the following formula (1), wherein L is a skin thickness and Td is a lag-time.

$$D=L^2/Td \qquad \text{Formula (1)}$$

In addition, here, the skin thickness L is an actual value by measurement of a human skin (the abdomen) provided for the test using a micrometer or the like.

Further, the lag-time is a node when extrapolating a straight line portion (steady state) in an accumulated penetration amount-time curve toward the time axis.

The distribution coefficient between base/skin of Bisoprolol in the patch of the invention is 0.8-2.0, preferably 1.0-2.0, and moreover preferably 1.0-1.5 from the viewpoint that Bisoprolol effective for therapy is distributed into body through the skin without exhibiting skin irritation.

Here, the distribution coefficient between base/skin K can be obtained by the following formula (2), wherein D is a skin diffusion coefficient, Cv is a drug concentration in a base, L is a skin thickness and J is a skin penetration rate at the steady state.

$$K=J \times L/D/Cv \qquad \text{Formula (2)}$$

In addition, here, the drug concentration in a base Cv is an actual concentration by previous measurement of a drug concentration contained in a formulation before the test starts.

Further, the skin penetration rate in the steady state is a slope of a straight line portion (steady state) in an accumulated penetration amount-time curve.

As to the claimed patch containing a backing and a Bisoprolol-containing pressure-sensitive adhesive layer comprising a base, wherein the Bisoprolol are contained in 10-15 mass % based on the total amount of the base, wherein the base comprises an acrylic polymer, and also wherein the base comprises one or more selected from, a rubber polymer, acrylic and silicon type polymers could also be used as a pressure-sensitive adhesive composition; however, a hydrophobic polymer is preferable, and moreover, the acrylic polymer and/or the silicon type polymer are preferably used to rise solubility of the active substance.

As the acrylic polymer, there is no particular restriction if it is a polymerized substance or a copolymerized substance containing at least one of (meth)acrylic derivatives represented by 2-ethylhexyl acrylate, methyl acrylate, butyl acrylate, hydroxyethyl acrylate, 2-ethylhexyl methacrylate and the like as a monomer unit; however, for example, adhesive polymers such as acrylic acid·octyl acrylate copolymer, 2-ethylhexyl acrylate·N-vinyl-2-pyrrolidone. 1,6-hexaneglycol dimethacrylate copolymer, 2-ethylhexyl acrylate·vinyl acetate copolymer, 2-ethylhexyl acrylate·vinyl acetate·acrylic acid copolymer, 2-ethylhexyl acrylate·2-ethylhexyl methacrylate·dodecyl methacrylate copolymer, methyl acrylate·2-ethylhexyl acrylate copolymerization resin emulsion and acrylic polymer contained in acryl resin alkanol amine liquid, Duro-Tak acrylic adhesive polymer series (manufactured by National Starch & Chemicals Company), GLEVA acryl adhesive polymer series (manufactured by Monsanto Co., Ltd.), Eudragit series (Higuchi Syoukai Co., Ltd.) and the like, can be used.

In addition, the acrylic polymer is preferably copolymer containing 2-ethylhexyl acrylate. As the copolymer, one containing an acrylic acid having carboxyl group in the molecule is more preferable, and one having substantially no alcoholic hydroxyl group in the molecule for the sake of further stability of the active substance is most preferable as the pressure-sensitive adhesive layer of the patch of the invention.

Using copolymer containing the acrylic acid having carboxyl group in the molecule is favorable because a pressure-sensitive adhesive force can be increased, and if required, it can be used as a reaction site in case of carrying out cross-linking.

Further, by use of an acrylic polymer which contains substantially no alcoholic hydroxyl group in the molecule, the formulation stability of Bisoprolol (or its salt) can be improved, while the reason is not sure, however, in case of presence of the alcoholic hydroxyl group it is anticipated that it is due to appearance of some interaction between the alcoholic hydroxyl group and Bisoprolol.

As such acrylic polymer, it is not particularly limited if it has substantially no alcoholic hydroxyl group and has carboxyl group, illustrative are, for example, 2-ethylhexyl acrylate·vinyl acetate·acrylic acid copolymer, Duro-Tak87-2852, Duro-Tak87-2194, Duro-Tak87-2196, Duro-Tak87-2353, Duro-Tak87-2051, Duro-Tak87-2052, Duro-Tak87-2054, Duro-Tak87-2825, Duro-Tak87-2677 (manufactured by National Starch & Chemicals Company) and the like.

Further, in a preparation step of the above acrylic polymer, when in the starting monomer, a monomer having hydroxyl group exists in a minute amount as impurities, or a side reaction such as thermal degradation occurs in case of polymerization, there is a case that the hydroxyl group resulting from the impurities is introduced in an obtained acrylic polymer, however, such acrylic polymer is comprised in the acrylic polymer having substantially no alcoholic hydroxyl group in the molecule and having carboxyl group so far as it does not impair features which the adhesive patch of the invention has.

In addition, as a hydrophobic polymer related to the invention, one which further contains a rubber polymer is preferable. By further containing the rubber polymer in the pressure-sensitive adhesive layer, the adhesiveness of a formulation can be controlled. The rubber polymer used in the invention contains both of a natural elastic polymer or a synthetic elastic polymer. Preferable examples of such rubber polymer include styrene-isoprene-styrene block copolymer, isoprene rubber, polyisobutylene, styrene-butadine-styrene block copolymer, styrene-butadine rubber, polysiloxane and the like. Among these, styrene-isoprene-styrene block copolymer and/or polyisobutylene are preferably used from the viewpoint of skin penetration of the drug.

Although the hydrophobic polymer may be used in one kind or by mix of two or more kinds, mix of the acrylic polymer and the rubber polymer is further preferable because a formulation satisfying both of skin penetration of the drug and physical properties of the formulation is given. Considering formation of a pressure-sensitive adhesive layer and a sufficient penetration, the blend amount of these polymers based on the weight of the total composition may be 5-90 mass %, preferably 10-70 mass %, more preferably 30-70 mass %.

In the invention it is desired to let an organic acid be contained in the pressure-sensitive adhesive layer in a case that the form of an active substance is a pharmaceutically acceptable acid-addition salt, and as organic acids used, illustrative are aliphatic (mono-, di-, tri-)carboxylic acids (e.g., acetic acid, propionic acid, isobutylic acid, caproic acid, caprylic acid, lactic acid, maleic acid, pyruvic acid, oxalic acid, succinic acid, tartaric acid and the like), aromatic carboxylic acids (e.g., phthalic acid, salicylic acid, bezoic acid, acetyl salicylic acid and the like), alkyl sulfonic acids (e.g., methanesulfonic acid, ethanesulfonic acid, propyl sulfonic acid, butanesulfonic acid, polyoxyethylene alkyl ether sulfonic acid and the like), alkyl sulfonic acid derivatives (e.g., N-2-hydroxyethyl-piperidine-N'-2-ethane sulfonic acid (hereinafter abbreviated as HEPES) and the like) and cholic acid derivatives(e.g., dehydrocholic acid and the like).

An absorption promoter may be contained in the pressure-sensitive adhesive layer of the patch of the invention, and as the absorption promoter, any compound in which an absorption promoting effect is shown may be used. Examples includes $C_6$-$C_{20}$ fatty acids, fatty alcohols, fatty acid esters, amides or ethers, aromatic organic acids, aromatic alcohols, aromatic fatty acid esters or ethers (these may be saturated or unsaturated, and may be cyclic, straight or branched), furthermore lactic acid esters, acetic acid esters, monoterpene compounds, sesquiterpene compounds, Azone, Azone derivatives, pirotiodecane, glycerol fatty acid esters, propylene glycol fatty acid esters, sorbitan fatty acid esters (Span type), polysorbates (Tween type), polyethylene glycol fatty acid esters, polyoxyethylene hardened castor oils (HCO type), polyoxyethylene alkyl ethers, sucrose fatty acid esters, plant oils and the like.

Specifically, caprylic acid, capric acid, caproic acid, lauric acid, myristic acid, palmitic acid, stearic acid, isostearic acid, oleic acid, linoleic acid, linolenic acid, lauryl alcohol, myristyl alcohol, oleyl alcohol, isostearyl alcohol, cetyl alcohol, methy laurate, hexyl laurate, diethyl sebacate, lauric acid diethanolamide, isopropyl myristate, myristyl myristate, octyldecyl myristate, cetyl palmitate, salicylic acid, methyl salicylate, ethylene glycol salicylate, cinnamic acid, methyl cinnamate, cresol, cetyl lactate, lauryl lactate, ethyl acetate, propyl acetate, geraniol, thymol, eugenol, terpineol, 1-menthol, borneol, d-limonene, isoeugenol, isoborneol, nerol, dl-camphor, glycerol monocaprylate, glycerol monocaprate, glycerol monolaurate, glycerol monooleate, sorbitan monolaurate, sucrose monolaurate, polysorbate 20, propylene glycol, propylene glycol monolaurate, polyethylene glycol monolaurate, polyethylene glycol monostearate, polyoxyethylene lauryl ether, HCO-60, pyrothiodecane and olive oil are preferable, and lauryl alcohol, myristyl alcohol, oleyl alcohol, isostearyl alcohol, diethyl sebacate, lauric acid diethanolamide, isopropyl myristate, glycerol monocaprate, glycerol monolaurate, glycerol monooleate, sorbitan monolaurate, propylene glycol monolaurate, polyoxyethylene lauryl ether and pyrothiodecane are preferable.

Such absorption promoter may be used by mix of two or more kinds, and considering a sufficient penetration as the patch, irritation to the skin such as rubor, edema, etc., and the like, it can be mixed preferably in 0.01-40 mass % based on the weight of the total composition of the pressure-sensitive adhesive layer, more preferably 0.05-30 mass %, in particular preferably 0.1-20 mass %.

A plasticizer may be contained in the pressure-sensitive adhesive layer of the patch of the invention. As usable plasticizers, illustrative are petroleum oils (e.g., paraffin type process oil, naphthalene type process oil, aromatic type process oil and the like), squalane, squalene, vegetable oils (e.g., olive oil, camellia oil, castor oil, tall oil, peanut oil), silicone oil, dibasic acid esters (e.g., dibutyl phthalate, dioctyl phthalate and the like), liquefied rubber (e.g., polybutene, liquefied isoprene rubber), liquefied fatty acid esters (isopropyl myristate, hexyl laurate, diethyl sebacate, diisopropyl sebacate), diethylene glycol, polyethylene glycol, glycol salicylate, propylene glycol, dipropylene glycol, triacetin, triethyl citrate, crotamiton and the like. In particular, liquid paraffin, liquefied polybutene, isopropyl myristate, diethyl sebacate and hexyl laurate are preferable.

These ingredients may be used by mix of two or more kinds, and considering a sufficient penetration and maintenance of a sufficient agglutinative force as the patch, the mix amount of such plasticizers based on the total composition in the pressure-sensitive adhesive layer can be 1-70 mass % in total, preferably 3-50 mass %, more preferably 5-40 mass %.

A tackifying resin may desirably be contained in the pressure-sensitive adhesive layer of the invention in case of lacking in an adhesive force to make the application possible for at least 12 hours , and as usable tackifying resins, illustrative are rosin derivatives (e.g., rosin, glycerol esters of rosin, hydrogenated rosin, glycerol esters of hydrogenated rosin, pentaerythritol esters of rosin and the like), alicyclic saturated hydrocarbon resins (e.g., Arcon P 100, Arakawa Chemical Industries, Co., Ltd.), aliphatic hydrocarbon resins (e.g., Quintone B 170, Zeon Corporation), terpene resins (e.g., Clearon P-125, Yasuhara Chemical), maleic acid resins and the like. In particular, glycerol esters of hydrogenated rosin, alicyclic saturated hydrocarbon resins and terpene resins are preferable.

Considering a sufficient adhesive force as the patch and irritation to the skin at the time of dissection, the mix amount of such tackifying resins based on the total composition in the pressure-sensitive adhesive layer can be 1-70 mass %, preferably 5-60 mass %, more preferably 10-50 mass %.

In addition, as required, antioxidants, fillers, cross-linking agents, preservatives or UV absorbers can be used. As antioxidants, tocopherol and its ester derivatives, ascorbic acid, ascorbic acid-stearic acid ester, nordihydroguaretic acid, dibutyl hydroxy toluene (BHT), butyl hyroxy anisole and the like are desirable. As fillers, calcium carbonate, magnesium carbonate, silicate (e.g., aluminum silicate, magnesium silicate and the like), silicic acid, barium sulfate, calcium sulfate, calcium zincate, zinc oxide, titanic oxide and the like are desirable. As cross-linking agents, thermosetting resins such as amino resins, phenol resins, epoxy resins, alkyd resins and unsaturated polyesters, isocyanate compounds, block isocyanate compounds, organic type cross-linking agents, and inorganic type cross-linking agents such as metals or metal compounds, are desirable. As preservatives, ethyl p-hydroxy benzoate, propyl p-hydroxy benzoate, butyl p-hydroxy benzoate and the like are desirable. As UV absorbers, p-amino benzoic acid derivatives, anthranilic acid derivatives, salicylic acid derivatives, coumarin derivatives, amino acid type compounds, imidazoline derivatives, pyrimidine derivatives, dioxane derivatives and the like are desirable.

Such antioxidants, fillers, cross-linking agents, preservatives and UV absorbers can be mixed preferably with an amount of not more than 10 mass % in total based on the weight of the total composition in the pressure-sensitive adhesive layer of the patch, more preferably not more than 5 mass % and in particular preferably not more than 2 mass %.

The drug-containing pressure-sensitive adhesive layer containing the composition described above can be prepared by any method. For example, a base composition containing the drug is heat-melted, coated on a removable paper or a backing, followed by affixing to the backing or the removable paper to give the present formulations. In addition, base components containing the drug are dissolved in solvent such as toluene, hexane or ethyl acetate, spreaded on the removable paper or the backing, dried to remove solvent, followed by affixing to the backing or the removable paper to give the present formulations. In addition, the thickness of an adhesive mass in the adhesive layer of the invention is 10-300μm, preferably 25-200μm, and more preferably 50-150μm considering a sufficient penetration and a sufficient pressure-sensitive adhesive force as the patch.

Although the patch of the invention is typically a patch shown in Fig. 6, as to the backing, an elastic or a non-elastic backing can be used. For example, it can be selected from a woven fabric, a knitted fabric, a nonwoven fabric, polyurethane, polyester, polyvinyl acetate, polyvinylidene chloride, polyethylene, polyethylene terephthalate, an aluminum sheet, etc., or composite materials thereof.

In addition, although the liner is not particularly limited if it can protect the pressure-sensitive adhesive layer till applying the patch to the skin, specifically, films such as polyesters (polyethylene terephthalate, etc.), polyvinyl chloride and polyvinylidene chloride, a laminated film of a high-quality paper with polyolefin, and the like may be illustrated. In these liners, if a silicone treatment is applied to the surface of the side attached to the pressure-sensitive adhesive layer, it is preferable because operation easiness in case of releasing the liner from the formulation is facilitated. Further, the formulation area in the patch of the invention is 1-60cm$^2$, preferably 1-40cm$^2$, more preferably 1-20cm$^2$ considering an absorption amount of the drug.

[Example]

In the following, the invention is explained in more detail by the examples. Further, in the examples, "%" is mass % in all.

(Example 1)

| 2-Ethylhexyl acrylate·vinyl acetate·acrylic acid copolymer | |
|---|---|
| | 18.5% |
| Styrene-isoprene-styrene block copolymer | 8.0% |
| Alicyclic saturated hydrocarbon resin | 42.0% |
| Liquid paraffin | 10.5% |
| Diethyl sebacate | 7.0% |
| Sodium acetate | 4.5% |
| Bisoprolol hemifumarate | 10.0% |

(Thickness of adhesive mass: 100$\mu$m)

Bisoprolol hemifumarate, sodium acetate, liquid paraffin and diethyl sebacate were put in a mortar and mixed thoroughly . The mixture was mixed with a solution in which 2-ethylhexyl acrylate·vinyl acetate·acrylic acid copolymer, styrene-isoprene-styrene block copolymer and alicyclic saturated hydrocarbon resin (Arcon P 100, manufactured by Arakawa Chemical Industries, Co., Ltd.) were dissolved in toluene and ethyl acetate to obtain a coating solution. Further, the mix ratio of each ingredient is as shown in the above formula.
Then, after the coating solution obtained was coated on a removable film made by polyethylene terephthalate, toluene and ethyl acetate of solvent were removed by drying to form a pressure-sensitive adhesive layer having a designated thickness of the adhesive mass. Further, by affixing the pressure-sensitive layer to a backing made by polyethylene terephthalate a patch of the invention was obtained.

(Example 2)

| 2-Ethylhexyl acrylate·vinyl acetate·acrylic acid copolymer | |
|---|---|
| | 68.6% |
| Isopropyl myristate | 10.0% |
| Sodium acetate | 6.4% |
| Bisoprolol hemifumarate | 15.0% |

(Thickness of adhesive mass: 100$\mu$m)

Bisoprolol hemifimarate, sodium acetate and isopropyl myristate were put in a mortar and mixed thoroughly. The mixture was mixed with a solution in which 2-ethylhexyl acrylate·vinyl acetate·acrylic acid copolymer was dissolved in heptane and ethyl acetate to obtain a coating solution. Further, the mix ratio of each ingredient is as shown in the above formula.
Then, after the coating solution obtained was coated on a removable film made by polyethylene terephthalate, heptane and ethyl acetate of solvent were removed by drying to form a pressure-sensitive adhesive layer having a designated thickness of the adhesive mass. Further, by affixing the pressure-sensitive layer to a backing made by polyethylene terephthalate a patch of the invention was obtained.

(Comparative example 1)

| 2-Ethylhexyl acrylate·vinyl acetate·acrylic acid copolymer | |
|---|---|
| | 42.2% |
| Propylene glycol monolaurate | 10% |
| Sodium acetate | 12.8% |
| Bisoprolol hemifumarate | 30% |

(Thickness of adhesive mass: 120μm)

A patch of the invention was obtained according to the above formula.

(Comparative example 2)

| 2-Ethylhexyl acrylate·vinyl acetate·acrylic acid copolymer | |
|---|---|
| | 52.2% |
| Isopropyl myristate | 3% |
| Sodium acetate | 12.8% |
| Bisoprolol hemifumarate | 30% |

(Thickness of adhesive mass: 120μm)

A patch of the invention was obtained according to the above formula.

(Comparative example 3)

| 2-Ethylhexyl methacrylate·dodecyl methacrylate·2-ethylhexyl | |
|---|---|
| acrylate copolymer | 79.4% |
| Isopropyl palmitate | 10.0% |
| Sodium acetate | 2.6% |
| Bisoprolol hemifumarate | 8.0% |

(Thickness of adhesive mass: 50μm)

A patch of the invention was obtained according to the above formula.

Test 1: Human skin penetration test

A human skin (the abdomen) for test use, which was removed in thickness of about 500 μm from the skin's cornified-layer side, was used. Each formulation (5cm$^2$) obtained in the examples and the comparative examples was attached to the skin's cornified-layer side and the dermal side was placed to the receptor-layer side to mount the skin on a flow-through type diffusion cell. Further, using pH7.4 phosphate buffer saline in the receptor layer, warm water was circulated around the outer part to make the temperature of the skin surface 32±1°C. The flow rate was made 5mL/hour and samplings were carried out at every 2 hours. As to a receptor solution obtained at each time, the flow volume was accurately measured, the drug concentration was measured by high-performance liquid chromatography (see Fig. 5 showing an accumulated penetration of Bisoprolol·time curve when successively administering the patch of the invention). The penetration rate per hour was calculated by measured values of the flow volume and the drug concentration, and the maximum skin penetration rate of each of the examples and the comparative examples was determined. Further, the diffusion coefficient D and the distribution coefficient K were calculated by the following formula (1) and formula (2) respectively.

$$D=L^2/Td \qquad \text{Formula (1)}$$

$$K=J\times L/D/Cv \qquad \text{Formula (2)}$$

Further, the symbols represent the following meanings.

D: Diffusion coefficient
L: Skin thickness
Td: Lag time
K: Distribution coefficient between base/skin
J: Skin penetration rate at steady state
Cv: Drug concentration in base

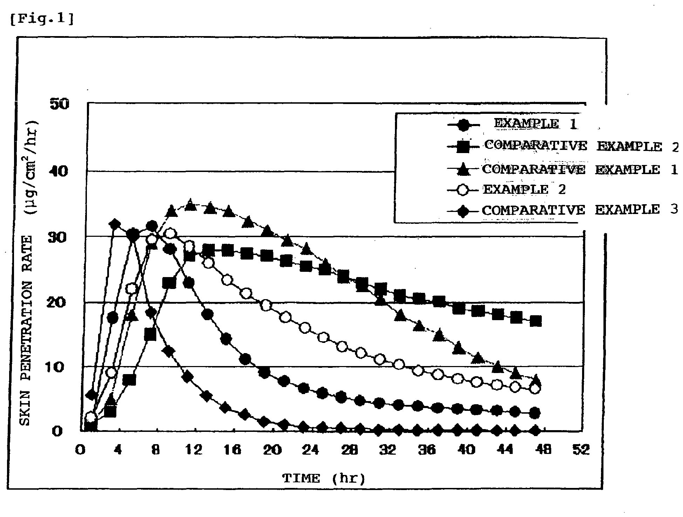

The results are shown in Table 1 and Fig.1.

(Result)

[Table 1]

Table 1

| | Maximum skin penetration rate $J_{max}$ [$\mu$g/cm$^2$/h] | Distribution coefficient [-] | Diffusion coefficient [x10E-8cm$^2$/sec] | Skin penetration rate after 24 hrs $J_{24}$ [$\mu$g/cm$^2$/h] | $J_{24}/J_{max}$ |
|---|---|---|---|---|---|
| Example 1 | 31.6 | 1,13 | 4.36 | 6.73 | 0.21 |
| Example 2 | 30.6 | 0.88 | 3.12 | 16.0 | 0.52 |
| Comparative example 1 | 35.0 | 0.69 | 2.34 | 28.2 | 0.81 |
| Comparative example 2 | 28.0 | 0.73 | 1.80 | 25.5 | 0.91 |
| Comparative example 3 | 32.0 | 0.75 | 7.24 | 0.70 | 0.02 |

As the results of the test, the formulations of the examples 1 and 2 showed the behavior that after the skin penetration rate became the maximum level within 6-12 hours, it gradually decreased.
As for the formulations of the comparative examples 1 and 2, after the skin penetration rate showed the maximum level, it maintained the steady state.
As for the formulation of the comparative example 3, after the skin penetration rate showed the maximum level, it rapidly decreased resulting to a state that the drug did not penetrate.

Test 2: Calculation method for human blood plasma concentration profile

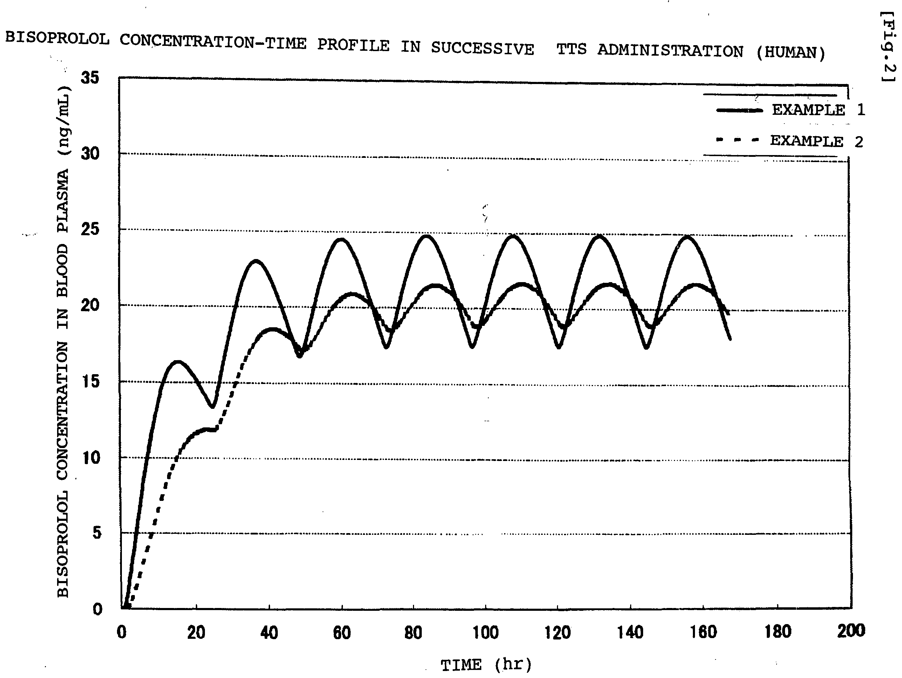

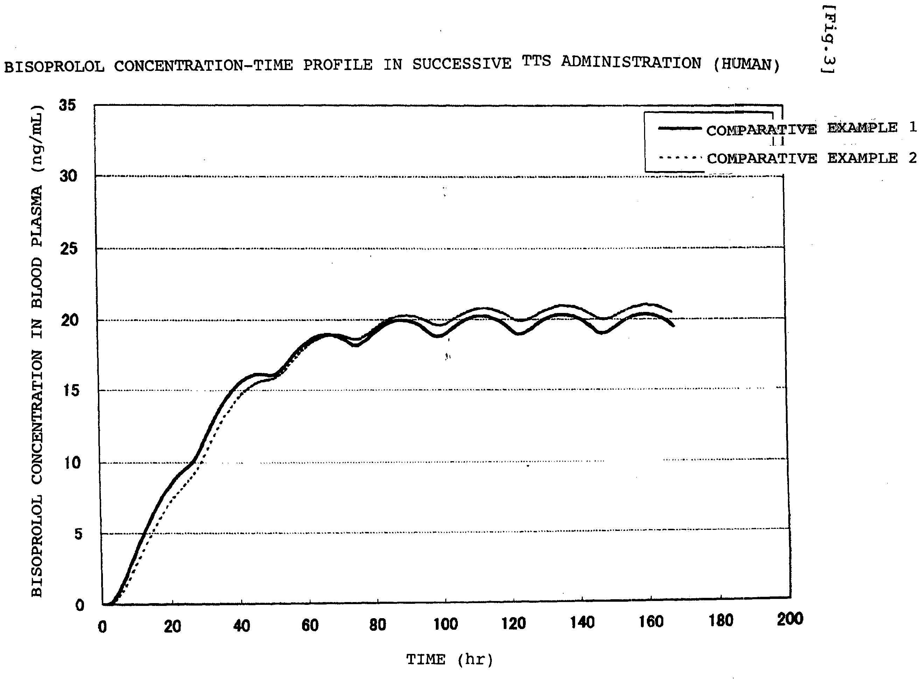

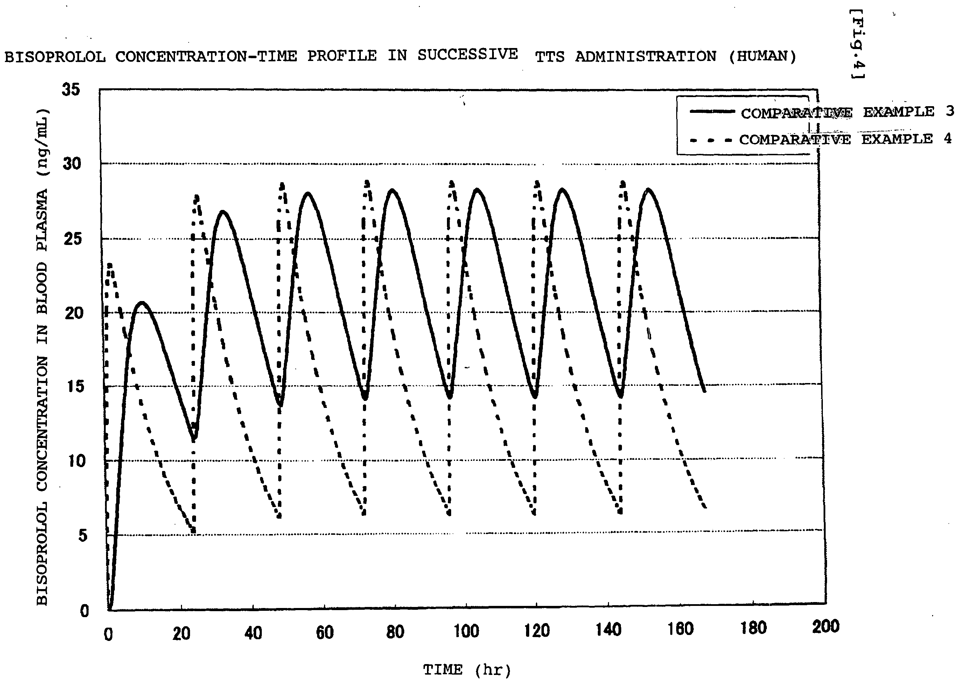

A pharmacodynamic parameter in a human oral administration of Bisoprolol hemifumarate was determined by WinNonlin (Scientific Consulting Inc.), a pharmacodynamics analysis soft, using known data of oral formulations (5mg). Human blood plasma concentrations at the time of single administration and at the time of successive administration were calculated by SKIN-CADTM Professional Edition ver.3.0 (i-HIVE Communication Inc.), a percutaneous absorption estimation system, using those parameters and the test results of the human skin penetration (Fig.1) obtained in the example 1 and 2. The results are shown in Fig.2-4. Further, a formulation area was made 15cm$^2$ in the example 1, 13cm$^2$ in the example 2, 7cm$^2$ in the comparative example 1, 8cm$^2$ in the comparative example 2, and 33cm$^2$ in the comparative example 3 respectively. In addition, for comparison, the concentration in blood plasma for 5-mg oral formulation was shown together.

As for the formulations of the examples 1 and 2, the Bisoprolol concentration in blood plasma reached to the steady state in shorter period even in case of carrying out the successive administration, whereby a stable formulation was obtained which showed a little difference between the maximum level and the minimum level and a little fluctuations. Namely, it is possible to make Bisoprolol patch in which a stable drug effect appears quickly and side effects are scarce.

As for the formulations of the comparative examples 1 and 2, although the Bisoprolol concentration in blood plasma showed a little difference between the maximum level and the minimum level, it take 3-4 days to reach to the steady state. Although these formulations are few in side effects, they are Bisoprolol patches which require much time until a stable drug effect appears.

As for the formulations of the comparative examples 3 and the comparative example 4 (oral formulation), the Bisoprolol concentration in blood plasma showed a big difference between the maximum level and the minimum level, and no stable formulation could be obtained. In these formulations, no drug effect appears during the time zone around the minimum level, and around the maximum level it is feared that side effects such as bradycardia and dizziness appear due to an excessive hypertension.

Test 3: Skin irritation test

As one of criteria to evaluate the safety of Bisoprolol patch of the invention, a skin irritation test was carried out. The skin pH value and the primary skin irritation index were obtained by the following test method. The results are shown in the following.

[Test method]

The formulations ($3cm^2$) of the above examples 1 and 2, comparative example 1 and the reference example 1 were submitted to the test. The formulation was stuck on the back skin of a Japanese White rabbit (female, 6 rabbits per group) for 24 hours and then peeled off. According to the below Draize method, the skin primary irritation index (P.I.I) was calculated by the mean value in which erythema scores and edema scores after 1 hour and 48 hours were added.

(Evaluation standard and grade)

Erythema and eschar formation

No erythema: 0
Very slight erythema: 1
Well-defined erythema: 2
Moderate to severe erythema: 3
Severe erythema to slight eschar formation: 4

Edema formation

No edema: 0
Very slight edema: 1
Slight edema: 2
Moderate edema: 3
Severe edema: 4

P.I.I value

0-2.0: Slightly irritation
2.0-6.0: Moderate irritation
6.0-8.0: Severe irritation

Further, as to the skin without sticking of the formulation and the skin just after peeling off the formulation, the surface pH was measured.

(Result)

[Table 2]

| Table 2: Skin pH and skin primary irritation of sticking part formulation (N=5) | | |
|---|---|---|
| | Skin pH value | Skin primary irritation index |
| No application of formulation | 5.74 | - |
| Example 1 | 7.06 | 1.4 |
| Example 2 | 7.35 | 1.5 |
| Comparative example 1 | 7.76 | 2.4 |
| Reference example (Placebo formulation) | 6.00 | 1.3 |

From the results of Table 2, in the placebo formulation containing no Bisoprolol compound, although the skin surface pH of the sticking part rose slightly compared with the pH value before sticking of the formulation, the skin primary irritation was hardly observed.

In the case that the patch of the comparative example 1 containing Bisoprolol compound, the apparent increase of the skin surface pH occurred due to the basicity of the drug itself when a high skin penetration rate continued for long time, and as the result, it was observed that the skin primary irritation occurred easily. In the meantime, in case of administering the patches of the example 1 and 2, although the pH values rose slightly, the skin primary irritation was hardly observed.

[Brief Description of Drawing]

Figure 1 is the drawing indicating a human skin penetration rate when sticking patches of the invention.
Figure 2 is the drawing of a Bisoprolol blood plasma concentration change when successively administering patches of the invention.
Figure 3 is the drawing of a Bisoprolol blood plasma concentration change when successively administering patches of the invention.
Figure 4 is the drawing of a Bisoprolol blood plasma concentration change when successively administering patches of the invention.
Figure 5 is the drawing indicating an accumulated penetration of Bisoprolol·time curve when successively administering patches of the invention.
Figure 6 is the drawing indicating the structure of a patch of the invention.

[Explanation of Numerals]

1···    BAKING FILM LAYER

2...    DRUG CONTAINING LAYER

3···    LINER

Claims

1.   A patch containing a backing and a Bisoprolol-containing pressure-sensitive adhesive layer comprising a base, wherein the Bisoprolol and/or a pharmaceutically acceptable salt thereof are contained in 10-15 mass % based on the total amount of the base, wherein the base comprises an acrylic polymer, and wherein a skin penetration rate of Bisoprolol after 24 hours is 15 to 60% of a maximum skin penetration rate thereof.

2.  The patch according to claim 1, wherein the maximum skin penetration rate of Bisoprolol is obtained within 12 hours during administration.

3.  The patch according to claims 1 or 2, wherein a skin diffusion coefficient of Bisoprolol is $2.5\times10E-8\sim6\times10E-8$ cm$^2$/sec.

4.  The patch according to any one of claims 1 to 3, wherein said patch is for repeated administration of once a day.

5.  The patch according to any one of claims 1 to 4, wherein the base comprises one or more selected from a styrene-isoprene-styrene block copolymer, a polyisoprene, and a silicone type polymer.

6.  The patch according to any one of claims 1 to 5, wherein the base further comprises a rubber polymer.

7.  The patch according to claim 6, wherein the rubber polymer comprises a natural elastic polymer or a synthetic elastic polymer.

8.  The patch according to any one of claims 1 to 6, wherein the acrylic polymer has substantially no alcoholic hydroxyl group and has carboxyl group.

**Patentansprüche**

1.  Pflaster, enthaltend einen Träger und eine Bisoprolol enthaltende drucksensitive Klebeschicht, umfassend eine Basis, wobei das Bisoprolol und/oder ein pharmazeutisch akzeptierbares Salz davon in 10-15 Masse-%, basierend auf der Gesamtmenge der Basis, enthalten sind, wobei die Basis ein Acrylpolymer umfasst und wobei eine Hautpenetrationsrate von Bisoprolol nach 24 Stunden 15 bis 60% einer maximalen Hautpenetrationsrate davon beträgt.

2.  Pflaster nach Anspruch 1, wobei die maximale Hautpenetrationsrate von Bisoprolol innerhalb von 12 Stunden während der Anwendung erreicht wird.

3.  Pflaster nach Anspruch 1 oder 2, wobei ein Hautdiffusionskoeffizient von Bisoprolol 2,5x10E-8~6x10E-8 cm$^2$/sek. beträgt.

4.  Pflaster nach einem der Ansprüche 1 bis 3, wobei das Pflaster für wiederholte einmal tägliche Anwendung ist.

5.  Pflaster nach einem der Ansprüche 1 bis 4, wobei die Basis eines oder mehrere, ausgewählt aus einem Styrol-Isopren-Styrol Blockcopolymer, einem Polyisopren und einem Silikontyp-Polymer umfasst.

6.  Pflaster nach einem der Ansprüche 1 bis 5, wobei die Basis weiterhin ein Gummipolymer umfasst.

7.  Pflaster nach Anspruch 6, wobei das Gummipolymer ein natürliches elastisches Polymer oder ein synthetisches elastisches Polymer umfasst.

8.  Pflaster nach einem der Ansprüche 1 bis 6, wobei das Acrylpolymer im Wesentlichen keine alkoholische Hydroxylgruppe aufweist und eine Carboxygruppe aufweist.

**Revendications**

1.  Timbre contenant un support et une couche d'adhésif sensible à la pression contenant du bisoprolol comprenant une base, dans lequel le bisoprolol et/ou un sel pharmaceutiquement acceptable de celui-ci sont contenus dans 10 à 15 % en masse basés sur la quantité totale de la base, dans lequel la base comprend un polymère acrylique, et dans lequel un taux de pénétration cutanée du bisoprolol après 24 heures est de 15 à 60 % d'un taux de pénétration cutanée maximal de celui-ci.

**2.** Timbre selon la revendication 1, dans lequel le taux de pénétration cutanée maximal du bisoprolol est obtenu dans les 12 heures au cours de l'administration.

**3.** Timbre selon la revendication 1 ou 2, dans lequel un coefficient de diffusion cutanée du bisoprolol est de 2,5x10E-8~6x10E-8 cm$^2$/s.

**4.** Timbre selon l'une quelconque des revendications 1 à 3, dans lequel ledit timbre est destiné à l'administration répétée d'une fois par jour.

**5.** Timbre selon l'une quelconque des revendications 1 à 4, dans lequel la base comprend un ou plusieurs composés parmi un copolymère séquencé de styrène-isoprène-styrène, un polyisoprène et un polymère de type silicone.

**6.** Timbre selon l'une quelconque des revendications 1 à 5, dans lequel la base comprend en outre un polymère de caoutchouc.

**7.** Timbre selon la revendication 6, dans lequel le polymère de caoutchouc comprend un polymère élastique naturel ou un polymère élastique synthétique.

**8.** Timbre selon l'une quelconque des revendications 1 à 6, dans lequel le polymère acrylique n'a essentiellement aucun groupe hydroxyle alcoolique et a un groupe carboxyle.

[Fig.1]

[Fig.2]

BISOPROLOL CONCENTRATION-TIME PROFILE IN SUCCESSIVE TTS ADMINISTRATION (HUMAN)

EXAMPLE 1
EXAMPLE 2

BISOPROLOL CONCENTRATION IN BLOOD PLASMA (ng/mL)

TIME (hr)

[Fig.3]

BISOPROLOL CONCENTRATION-TIME PROFILE IN SUCCESSIVE TTS ADMINISTRATION (HUMAN)

EP 1 847 264 B1

[Fig.4]

BISOPROLOL CONCENTRATION-TIME PROFILE IN SUCCESSIVE TTS ADMINISTRATION (HUMAN)

———— COMPARATIVE EXAMPLE 3
- - - COMPARATIVE EXAMPLE 4

TIME (hr)

EP 1 847 264 B1

[Fig.5]

[Fig.6]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003313122 A
- EP 14622121 A
- WO 9518603 A
- JP 2004083523 B